(19) Europäisches Patentamt
European Patent Office
Office européen des brevets

(11) EP 1 900 361 B1

# (12) EUROPEAN PATENT SPECIFICATION

(45) Date of publication and mention of the grant of the patent:
**31.03.2010 Bulletin 2010/13**

(51) Int Cl.:
***A61K 31/14*** *(2006.01)* ***A61K 33/14*** *(2006.01)*
***A61K 38/48*** *(2006.01)*

(21) Application number: **07017057.6**

(22) Date of filing: **31.08.2007**

## (54) Solution for canal irrigation based on sodium hypochlorite and proteolytic enzyme

Auf Natriumhypochlorid und einem proteolytischen Enzym basierende Lösung zur Kanalbewässerung

Solution d'irrigation de canal basée sur une hypochlorite de sodium et enzyme protéolytique

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **08.09.2006 IT MI20061711**

(43) Date of publication of application:
**19.03.2008 Bulletin 2008/12**

(73) Proprietor: **Giovanni Ogna & Figli S.p.A.**
**20053 Taccona di Muggio MI (IT)**

(72) Inventor: **Taborelli, Corrado**
**20038 Seregno (MI) (IT)**

(74) Representative: **Pipparelli, Claudio**
**PIPPARELLI & PARTNERS**
**Via Quadronno, 6**
**20122 Milano (IT)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to the field of medical devices (according to dir. 93/42/CE) for the exclusive use of odontological doctors for endocanal treatment.

More specifically, the present invention relates to the use of an aqueous formulation comprising sodium hypochlorite, amine oxides and one or more enzymes selected from those having a proteolytic activity, as a lubricant and disinfectant for canal irrigation, having dispersing properties of the smear layer.

When the inner space of a tooth, containing the vital dental pulp, loses its insulation from the surrounding oral environment, it can be infiltrated by micro-organisms whose proliferation causes inflammation and progressive pulp degeneration with the consequent appearance of pain and other symptomatologies. It is therefore necessary to effect an endodontic intervention aimed at preserving the dental element which can still be recovered.

The main objective of endodontic treatment is the cleaning of the pulp chamber and of the infected endodontic radicular canals, their disinfection, shaping and subsequent sealing. The internal anatomy of teeth is complex and consists, in addition to a main canal for each root, of a series of canals and side openings.

The cleaning phase of the radicular canal system is consequently rather delicate due to the complex anatomy of the endodontic cavities, and requires the use of both mechanical instrumentation which mechanically removes the tissues, and irrigation liquids which facilitate the instrumentation action and are capable of penetrating all the branches of the radicular canal system, even in points which cannot be reached by mechanical instrumentation.

The purpose of the irrigation can therefore be summarized in the following points:

- Reduction in the bacterial charge present inside the radicular canal system;
- Dissolution of the necrotic tissues present;
- Lubrication of the canal instruments;
- Evacuation of the fragments caused by the mechanical instrumentation (smear layer)

It is currently impossible to achieve all the irrigation objectives in an optimal way using only one irrigator: present practice includes the alternating use of various solutions differing in their chemical nature and consequently in their activity, whose use globally achieves the objectives of the treatment.

Among the various chemical solutions used at present, sodium hypochlorite at different concentrations is certainly the most common and appreciated due to its interesting properties.

Sodium hypochlorite (CAS 8007-59-8) is a chemical substance which can be defined as sodium salt of the hypochlorous acid present in numerous chemical compounds of common use, among which bleach.

In the odontological field, it has been used since the forties' as an irrigating solution, by Walker who demonstrated the characteristic as solvent of organic substances and antiseptic within a wide range.

Its disinfecting property has been confirmed over the years: sodium hypochlorite can kill vegetative bacteria, spores, fungi, protozoan and viruses (including HIV, the viruses of hepatitis A and B).

In addition to a strong antimicrobial activity, sodium hypochlorite has a dissolvent activity towards organic and in particular necrotic tissues, present in infected radicular canals. This characteristic activity is specific of hypochlorite which other solutions used for canal irrigation do not have.

Both the antibacterial and the digestive activity of organic tissues can be incremented by heating the sodium hypochlorite solution, before application.

Sodium hypochlorite in solution also has a reasonable lubricating activity for the instruments.

It is known that hypochlorite has a poor dissolution activity of inorganic tissue, in particular of the so- called "smear layer". The latter, which is formed during the preparation of the canal, has a thickness ranging from 1 to 5 μm. This material, in addition to being deposited on the canal wall, can penetrate inside the tooth tubulus and consists of residues of dentine, pre-dentine, pulp residues, and bacteria when the tooth is infected. Normally, the bacteria are capable of surviving and multiply inside the smear layer, which can also hinder the antimicrobial action of the irrigating products and canal medications.

As mentioned above, in order to solve this problem, resort is normally made to the technique of alternate washings, i.e. before with a solution of sodium hypochlorite and after with solution capable of facilitating the removal of the smear layer.

This solution is obviously unsatisfactory, as the alternating use of two solutions is always complex and can carry various risks.

A solution based on sodium hypochlorite has now been found, which overcomes the above drawbacks.

According to this, the present invention relates to the use of an aqueous formulation as a lubricant and disinfectant for canal irrigation, having dispersing properties with respect to the smear layer, comprising:

a) sodium hypochlorite;

b) amine oxides having general formula

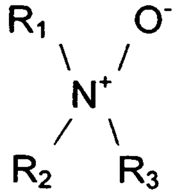

wherein $R_1$ and $R_2$, the same or different, are selected from methyl and ethyl; $R_3$ is an alkyl radical from $C_{10}$ to $C_{18}$, preferably from $C_{12}$ to $C_{16}$;

c) one or more enzymes selected from those having a proteolytic activity:

d) water.

The term disinfectant generally means a substance (or formulation) capable of killing, complexing or destroying bacteria in some way.

The term "dispersing property of the smear layer" means the capacity of the aqueous formulation of the present invention of removing and keeping the inorganic residues which form the "smear layer" dispersed in water.

The term "lubricant" means that the aqueous formulation of the present invention reduces the viscosity of the instruments during the intervention of the dentist.

Sodium hypochlorite is present in the aqueous formulation of the present invention in a quantity ranging from 0.5% to 10% as available chlorine, preferably from 2 to 8%, even more preferably from 4 to 6%. The amine oxide is present in a quantity ranging from 0.2 to 20% by weight, preferably from 1 to 10%, even more preferably from 4 to 5% by weight. The enzyme having a proteolytic property is present in a quantity ranging from 0.1 to 10% by weight, preferably from 0.8 to 6%, even more preferably from 1 to 2% by weight.

As far as the amine oxides are concerned, these are so-called amphoteric surfactants. Typical but non-limiting examples of these amphoteric surfactants are N,N-dimethyl dodecyl amine N-oxide, N,N-dimethyl tetradecyl amine N-oxide, N,N-dimethyl stearyl amine N-oxide, N,N-dimethyl decyl amine N-oxide, N,N-dimethyl cetyl amine N-oxide. Amine oxides can obviously be used in the present invention, in which $R_3$ is a mixture of $C_{10}$ to $C_{18}$ alkyls, preferably from $C_{12}$ to $C_{16}$. In the preferred embodiment the amine oxide is N,N-dimethyl dodecyl amine N-oxide (CAS[1643-20-5]).

As far as the enzymes having a proteolytic activity are concerned, these are commercially available (for example protease from Bacillus sp of Sigma, or Everlase® of Novozymes). The above enzymes have a proteolytic activity, i.e. they favour the lysis of proteins, molecules forming the organic pulp tissue. The above enzymes have the characteristic of resisting the extreme pH conditions of the sodium hypochlorite solution.

As far as the water is concerned, this is water purified through distillation to eliminate all possible substances which can alter the hypochlorite stability.

In the preferred embodiment, the composition of the present invention is heated to a temperature of 30 to 38°C, before use, preferably from 35 to 37°C. This has the purpose of increasing the efficacy.

The formulations of the present invention also have the property of being stable over time, if kept in a refrigerator (about 4 °C) and protected from light. The above-mentioned stability is determined by measuring the active chlorine variation.

In this respect, it should be noted that sodium hypochlorite is commercially available only in aqueous solutions having various titres; it is in fact synthesized by means of the reaction:

$$Cl_2 + 2\ NaOH \rightarrow NaOCl + NaCl + H_2O + heat$$

There are several ways of expressing the concentration of sodium hypochlorite: the most widely used is "available chlorine".

If we examine the following reactions:

$$NaOCl + 2\ KI + 2\ CH_3COOH \rightarrow I_2 + NaCl + 2\ CH_3COOK + H_2O$$

$$Cl_2 + 2\ KI \rightarrow I_2 + 2\ KCl$$

it can be seen that a molecule of sodium hypochlorite has the same oxidative capacity as a chlorine molecule, as both oxidize two molecules of potassium iodide.

The expression which connects the weight percentage of hypochlorite with the "available chlorine" is given by

the following relationship:

$$\text{wt \% NaOCl} = (\text{\% av.Cl}) \cdot \frac{\text{mol. wt NaOCl}}{\text{mol. wt } Cl_2} = (\text{\% av. Cl}) \cdot \frac{74.44}{70.91} = 1.05$$

therefore the weight % of NaOCl is obtained from the available chlorine % multiplied by 1.05.

As mentioned above, sodium hypochlorite in solution also has a reasonable lubricating activity for the instruments. The above-mentioned lubricating property is enhanced due to the presence of surfactants in the composition of the present invention.

The presence of surfactants in the formulation of the present invention also provides two more advantages: it decreases the surface tension of the product, favouring its penetration in points which cannot be reached by the instruments and it gives the product a suspension capacity for the fragments formed during the instrumentation.

Preliminary studies (see the experimental part) effected in parallel on sodium hypochlorite solutions and on the composition of the present invention, indicated with the same the concentration, a higher cleaning activity of the composition of the present invention, also with shorter treatment times.

**Product presentation**

As far as the usual application technique of sodium hypochlorite is concerned, all the preparations currently on the market present sodium hypochlorite in bottles, with an active chlorine titre ranging from 0.5 to 5.25%. The highest concentrations, over 2%, are corrosive for the tissues and this clinical datum is important, considering the operative sequence the doctor has to adopt:

- preparation of the operating table with dike and sucker
- opening of the flask seal
- preparation of a suitable syringe with needle
- insertion of the needle into the flask
- suction of the liquid
- treatment of the patient with the liquid.

This working sequence is particularly delicate as accidental spills of solution are possible, which can accidentally come into contact with the patient or dentist in particularly sensitive areas of the body, such as the eyes, with consequent irritation or even damage.

The moment in which the liquid is applied to the patient is of critical importance, but it is fundamental for a satisfactory result of the operation to have previously prepared the syringe and needle suitable for the canal irrigation. Also the manipulation of bottles, often made of glass, can involve the risk of spills or accidental falls, which can cause accidents.

A presentation of sodium hypochlorite in a syringe, with the relative needle which must be assembled by the dentist before using the product on the patient, is therefore of great interest.

The presentation of a syringe and needle in a kit can prevent possible accidents. It is in fact known that it is not necessary to exert an excessive force, or try to force the needle when a canal irrigation is effected with sodium hypochlorite. Even with all possible care, however, if a needle for injections is unintentionally used, it is highly probable that a radicular perforation or an apical leakage will occur.

The formulation of the present invention is parcelled in syringes, ready for use by the doctor, who simply has to insert a needle, preferably supplied in the package, with a blunted tip.

A further object of the present invention therefore relates to syringes for canal irrigation, made of inert material, preferably glass, containing the composition according to claim 1. The syringes are separately equipped with a washing needle made of steel (or plastic material) with a blunted tip.

The following examples are provided for a better understanding of the present invention.

**EXAMPLES**

In these examples, 1-dodecylamine, N,N-dimethyl-N-oxide was used as amine oxide, whereas the protease Bacillus Sub. of Sigma was used as proteolytic enzyme.

**Example 1**

| | |
|---|---|
| Sodium hypochlorite | 5% as active chlorine |
| Amine oxide | 4.5% |
| Proteolytic enzyme | 1.5% |
| Water | complement to 100 |

The preparation was obtained in the following way:

13.5 g of amine oxide (Laucosol ox, Industrie Chimiche Panzeri) were weighed in a 400 ml beaker. The pre-established quantity of water is added (50.7 g) and the whole composition is stirred in order to obtain a limpid and homogenous gel. Sodium hypochlorite is then added (226.8 g of a solution having a titre as active chlorine of 6.62%) and finally the enzyme. The composition is hand-stirred until complete homogenization.

A product with a very low viscosity is obtained (about 50-100 cP at 25°C, but homogeneous and stable, having a titre as active chlorine of 5.12 and a pH > 12.5.

**Example 2**

| | |
|---|---|
| Sodium hypochlorite | 3% as active chlorine |
| Amine oxide | 4.5% |
| Proteolytic enzyme | 1.5% |
| Water | complement to 100 |

The preparation of the product is the same that as described in example 1.
A product is obtained having a very low viscosity (about 20-50 cP at 25°C) homogeneous and stable, with a pH comparable with that of example 1 (>12.5)

**Evaluation of the removal capacity of the smear layer**

A preliminary study was carried out at the Insubria University, to evaluate the efficacy of the product of example 1 as a canal irrigating solution, having as base characteristics, those of hypochlorite, but with a higher aggressiveness with respect to the soft tissues of the endoduct and removal property of the smear layer. The need for a higher aggressiveness is due to the fact that modem mechanical canal instrumentation techniques require shorter exposure times of the endoduct to the irrigation solution.
The preliminary study included the exposure of two samples of extracted monoradicular teeth to different irrigating solutions, according to the following procedures:

*Reference sample A* (sodium hypochlorite) Fig. A

- manual and mechanical shaping with NiTi alternated with irrigations with sodium hypochlorite at 5.25% heated to 37°C;
- irrigation and maintaining the samples on hypochlorite for 1/2 hour;
- maintaining in a physiological solution;
- SEM vision treatment.

*Sample B with enzyme* (example composition) - Fig. B

- manual and mechanical shaping with NiTi alternated with irrigations, using the composition of example 1 heated to 37°C;
- maintaining in a physiological solution;
- SEM vision treatment.

Fig. A, obtained with SEM at 1200X, shows the presence of the smear layer along the wall of the endoduct and in the dental tubules.
Fig. B, obtained with SEM at 150X, shows the almost complete absence of the smear layer in the canal lumen and dental tubules.

The results of this preliminary study denote an optimum capacity of the composition of the present invention in the removal of the smear layer, also considering the short exposure to the irrigation solution with respect to the reference sample treated with sodium hypochlorite at 5.25%.

## Claims

1. Use of an aqueous formulation as a lubricant and disinfectant, for canal irrigation, having dispersing properties of the smear layer, comprising:

    a) sodium hypochlorite;
    b) amine oxides having general formula

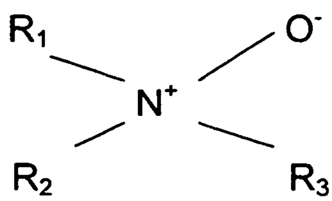

    wherein $R_1$ and $R_2$, the same or different, are selected from methyl and ethyl; $R_3$ is an alkyl radical from $C_{10}$ to $C_{18}$, preferably from $C_{12}$ to $C_{16}$;
    c) one or more enzymes selected from those having a proteolytic activity:
    d) water.

2. The use according to claim 1, wherein the aqueous solution comprises sodium hypochlorite in a quantity ranging from 0.5% to 10% as available chlorine, preferably from 2 to 8%, even more preferably from 4 to 6%.

3. The use according to claim 1 or 2, wherein the amine oxide is present in a quantity ranging from 0.2 to 20% by weight, preferably from 1 to 10% by weight, even more preferably from 4 to 5% by weight.

4. The use according to claims 1 to 3, wherein the enzyme having a proteolytic activity, is present in a quantity ranging from 0.1 to 10%, preferably from 0.8 to 6% by weight, even more preferably from 1 to 2% by weight.

5. The use according to claim 1, wherein the amine oxide is N,N-dimethyl dodecyl amine N-oxide.

6. The use according to claim 1, wherein the aqueous solution is heated before use to a temperature ranging from 30 to 38°C, preferably from 35 to 37°C.

7. Syringes for canal irrigation made of inert material, preferably glass, containing the formulation according to claim 1.

## Patentansprüche

1. Verwendung einer wässrigen Formulierung als Schmier- und Desinfektionsmittel zur Kanalbewässerung, mit dispergierenden Eigenschaften der Schmierschicht, umfassend:

    a) Natriumhypochlorid;
    b) Aminoxide einer allgemeinen Formel:

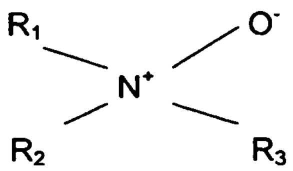

wobei $R_1$ und $R_2$, gleich oder verschieden, ausgewählt sind aus Methyl und Ethyl; $R_3$ ein Alkylrest mit 10 bis 18 Kohlenstoffatomen, vorzugsweise mit 12 bis 16 Kohlenstoffatomen ist;
c) ein oder mehrere Enzyme, ausgewählt aus solchen mit einer proteolytischen Aktivität;
d) Wasser.

**2.** Verwendung nach Anspruch 1, wobei die wässrige Lösung umfasst Natriumhypochlorid in einer Menge von 0,5% bis 10%, als verfügbares Chlor, vorzugsweise von 2 bis 8%, noch mehr bevorzugt von 4 bis 6%.

**3.** Verwendung nach Anspruch 1 oder 2, wobei das Aminoxid in einer Menge von 0,2 bis 20 Gew.-%, vorzugsweise von 1 bis 10 Gew.-%, noch mehr bevorzugt von 4 bis 5 Gew.-% vorliegt.

**4.** Verwendung nach Ansprüchen 1 bis 3, wobei das Enzym mit einer proteolytischen Aktivität in einer Menge von 0,1 bis 10%, vorzugsweise von 0,8 bis 6 Gew.-%, noch mehr bevorzugt von 1 bis 2 Gew.-% vorliegt.

**5.** Verwendung nach Anspruch 1, wobei das Aminoxid ein N,N-Dimethyldodecylamin-N-Oxid ist.

**6.** Verwendung nach Anspruch 1, wobei die wässrige Lösung vor der Verwendung auf eine Temperatur im Bereich von 30 bis 38°C, vorzugsweise von 35 bis 37 °C erwärmt wird.

**7.** Spritzen zur Kanalbewässerung aus einem inerten Material, vorzugsweise Glas, enthaltend die Formulierung nach Anspruch 1.

## Revendications

**1.** Utilisation d'une formulation aqueuse en tant que lubrifiant et désinfectant, pour l'irrigation canalaire, présentant des propriétés de dispersion de la boue dentinaire, comprenant :

a) de l'hypochlorite de sodium ;
b) des oxydes d'amine de formule générale

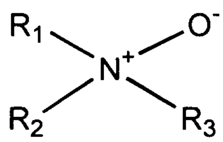

dans laquelle $R_1$ et $R_2$, qu'ils soient identiques ou différents, sont choisis parmi le méthyle et l'éthyle ; et dans laquelle $R_3$ est un radical alkyle compris entre $C_{10}$ et $C_{18}$, préférentiellement entre $C_{12}$ et $C_{16}$ ;
c) un ou plusieurs enzymes choisis parmi ceux présentant une activité protéolytique ; et
d) de l'eau.

**2.** Utilisation selon la revendication 1, dans laquelle la solution aqueuse comprend de l'hypochlorite de sodium en une quantité comprise entre 0,5% et 10% en chlore disponible, préférentiellement comprise entre 2% et 8% et encore plus préférentiellement comprise entre 5% et 6%.

**3.** Utilisation selon la revendication 1 ou 2, dans laquelle l'oxyde d'amine est présent en une quantité comprise entre

0,2% et 20% en poids, préférentiellement comprise entre 1 % et 10% en poids et encore plus préférentiellement comprise entre 4% et 5% en poids.

4. Utilisation selon les revendications 1 à 3, dans laquelle l'enzyme présentant une activité protéolytique est présente en une quantité comprise entre 0,1% et 10% en poids, préférentiellement comprise entre 0,8% et 6% en poids et encore plus préférentiellement comprise entre 1 % et 2% en poids.

5. Utilisation selon la revendication 1, dans laquelle l'oxyde d'amine est du N-oxyde de N,N-diméthyl-dodécylamine.

6. Utilisation selon la revendication 1, dans laquelle la solution aqueuse est chauffée avant utilisation à une température comprise entre 30 et 38°C, préférentiellement comprise entre 35 et 37°C.

7. Seringue pour l'irrigation canalaire fabriquée en un matériau inerte, préférentiellement du verre, contenant la formulation selon la revendication 1.

TABLE 1

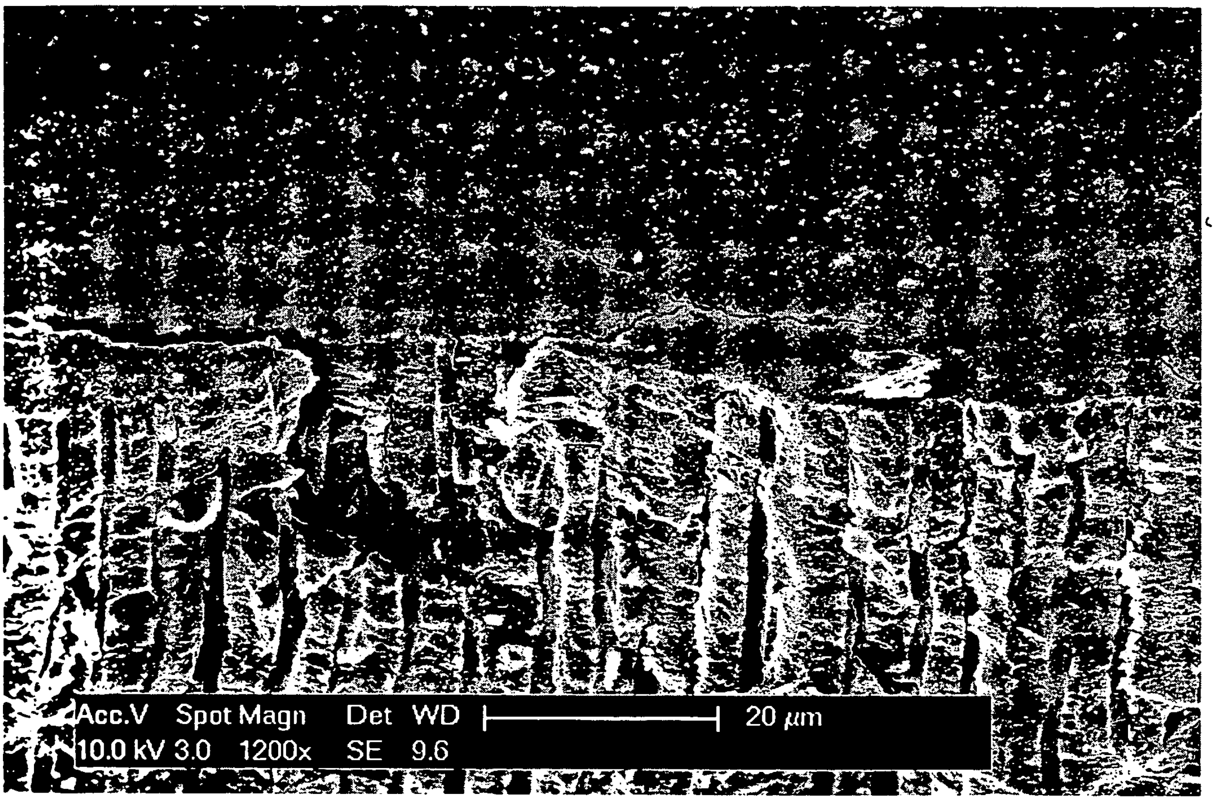


Fig. A

TABLE 2

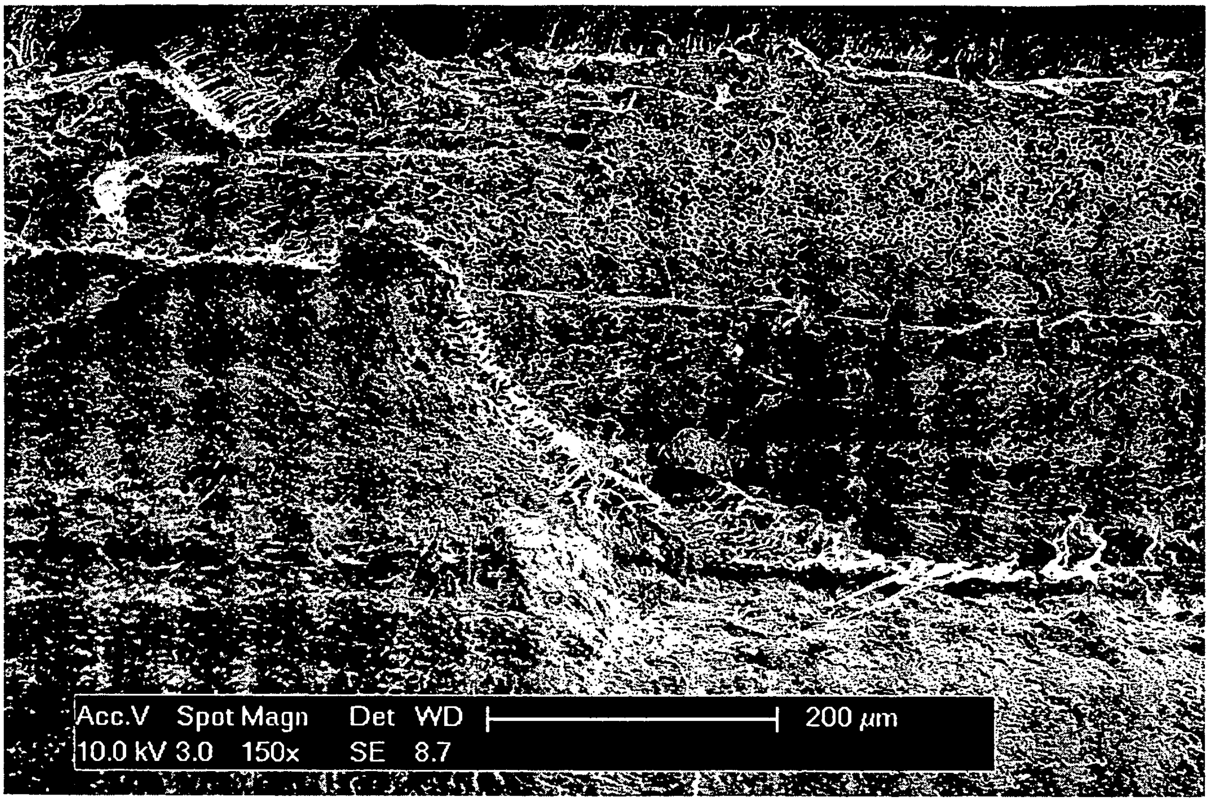


Fig. B